# EUROPÄISCHE PATENTSCHRIFT

(11) **EP 1 268 400 B1**
(45) Veröffentlichungstag und Bekanntmachung des Hinweises auf die Patenterteilung: **11.08.2004**
(21) Anmeldenummer: 01933703.9
(22) Anmeldetag: 15.03.2001
(51) Int. Cl.: C07C 231/00, C07C 233/54, C07C 233/15, C07C 231/12

(54) **VERFAHREN ZUR HERSTELLUNG VON N-BUTYRYL-4-AMINO-3-METHYL-BENZOESÄUREMETHYLESTER UND DIE NEUE VERBINDUNG N-(4-BROM-2-METHYLPHENYL)-BUTANAMID**
METHOD FOR PRODUCING N-BUTYRYL-4-AMINO-3-METHYL-METHYL BENZOATE AND THE NOVEL COMPOUND N-(4-BROMINE-2-METHYLPHENYL)-BUTANAMIDE
PROCEDE DE PREPARATION D'ESTER DE METHYLE D'ACIDE N-BUTYRYL-4-AMINO-3-METHYL-BENZOIQUE ET LE NOUVEAU COMPOSE N-(4-BROME-2-METHYLPHENYL)-BUTANAMIDE

(30) Priorität: 28.03.2000 DE 10015279
(43) Veröffentlichungstag der Anmeldung: 02.01.2003
(73) Patentinhaber: Bayer Chemicals AG, 51368 Leverkusen (DE)
(72) Erfinder: RODEFELD, Lars, 51375 Leverkusen (DE); HÖPFNER, Thomas, 40764 Langenfeld (DE); KLAUSENER, Alexander, 50259 Pulheim (DE); BEHRE, Horst, 51519 Odenthal (DE)
(86) Internationale Anmeldenummer: PCT/EP2001/002924
(87) Internationale Veröffentlichungsnummer: WO 2001/072690

(56) Entgegenhaltungen:
- EP-A- 0 675 102
- RIES U J ET AL: "6-Substituted benzimidazoles as new nonpeptide angiotensin II receptor antagonists: synthesis, biological activity, and structure-activity relationships" JOURNAL OF MEDICINAL CHEMISTRY, AMERICAN CHEMICAL SOCIETY. WASHINGTON, US, Bd. 36, Nr. 25, 1993, Seiten 4040-4051, XP002135628 ISSN: 0022-2623 in der Anmeldung erwähnt
- DATABASE CHEMCATS [Online] 19. Februar 2001 (2001-02-19) retrieved from STN Database accession no. 2001:1202716 XP002179987

## Beschreibung

Die vorliegende Erfindung betrifft ein verbessertes Verfahren zur Herstellung von N-Butyryl-4-amino-3-methyl-benzoesäuremethylester und die neue chemische Verbindung N-(4-Brom-2-methylphenyl)-butanamid.

4'-[[2-n-Propyl-4-methyl-6-(1-methylbenzimidazol-2-yl]-methyl]-biphenyl-2-carbonsäure ist ein wertvoller Angiotensin-Antagonist, insbesondere ein wertvoller Angiotensin-II-Antagonist (siehe EP-A 502 314). Im folgenden wird diese Carbonsäure auch kurz Antagonist genannt.

In J. Med. Chem. 1993, 4040 wird eine Synthese des Antagonisten beschrieben, bei der man von 4-Amino-3-methyl-benzoesäuremethylester (I) ausgeht, der mit Buttersäurechlorid zum N-Butyryl-4-amino-3-methyl-benzoesäuremethylester (II) umgesetzt wird (siehe folgendes Reaktionsschema (1)).

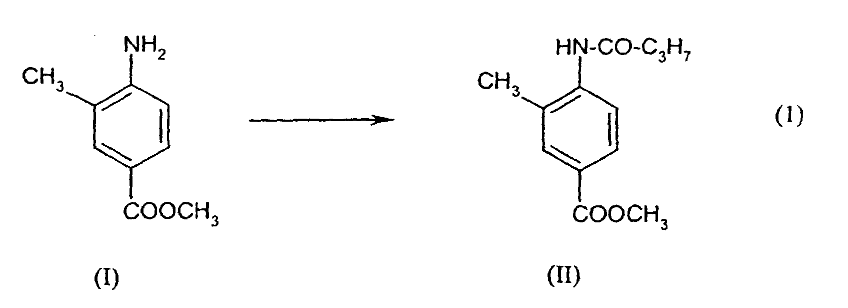

Die Verbindung (II) wird dann in weiteren Schritten zum Antagonisten umgesetzt.

Die benötigte Ausgangsverbindung der Formel (I) ist nur unvorteilhafter Weise zugänglich. So kann man von 4-Nitro-m-xylol (III) ausgehen, dieses durch Oxidation in 4-Nitro-2-methyl-benzoesäure (IV) überführen (siehe Liebigs Ann. Chem. 144, 163 (1867)), das man zum 4-Nitro-2-methyl-benzoesäuremethylester (V) verestert (siehe Chem. Ber. 102, 2502 (1969)) und diesen zum 4-Amino-3-methyl-benzoesäuremethylester reduziert (I) (siehe Chem. Ber. a.a.O.). Dieses Verfahren zur Herstellung der Verbindung (II) wird durch das folgende Reaktionsschema (2) illustriert.

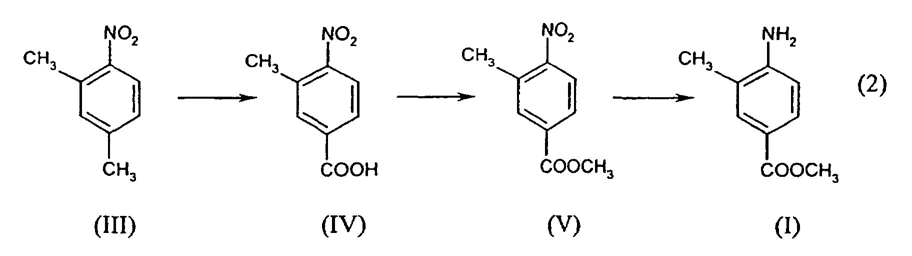

Wie ersichtlich besteht das bekannte Verfahren zur Herstellung der Verbindung (II) aus vier Einzelstufen, wobei die erste Stufe (III) → (IV) besonders nachteilig ist, weil sie lange Reaktionszeiten benötigt und sie nur unselektiv und deshalb mit niedrigen Ausbeuten zu (IV) führt. Gemäß J.O.C. 32, 134 (1967) wird eine Reaktionszeit von 20 Stunden benötigt und die Ausbeuten liegen bei 22,5 bis 27 %.

Es besteht deshalb immer noch das Bedürfnis nach einem Verfahren zur Herstellung der Verbindung (II), bei dem weniger Stufen notwendig sind und die Verbindung (II) in vorteilhafter Weise erhalten werden kann.

Es wurde nun ein Verfahren zur Herstellung von N-Butyryl-4-amino-3-methylbenzoesäuremethylester (II) gefunden, das dadurch gekennzeichnet ist, dass man o-Toluidin (VI) mit Buttersäurechlorid zu N-(2-Methylphenyl)-butanamid (VII) umsetzt, dieses zu N-(4-Brom-2-methylphenyl)-butanamid (VIII) bromiert und dieses durch Umsetzung mit Kohlenmonoxid und Methanol in Gegenwart eines Palladiumkatalysators in N-Butyryl-4-amino-3-methyl-benzoesäuremethylester (II) überführt. Das folgende Reaktionsschema (3) erläutert das erfindungsgemäße Verfahren.

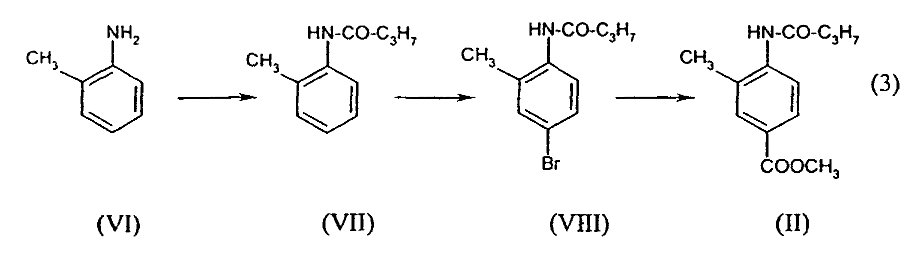

Die Stufen (VII) → (VIII) und (VIII) → (II) sind dabei besonders überraschend vorteilhaft, denn sie liefern (VIII) bzw. (II) jeweils in Ausbeuten von über 95 %.

Die erste Stufe des erfindungsgemäßen Verfahrens, die Umsetzung der Verbindung (VI) mit Buttersäurechlorid zur Verbindung (VII), kann beispielsweise durchgeführt werden, indem man die Verbindung (VI) in einem inerten Lösungsmittel, z.B. einem aromatischem Lösungsmittel wie Chlorbenzol, Toluol oder Xylol vorlegt und anschließend bei Temperaturen von beispielsweise 50 bis 100°C Buttersäurechlorid zudosiert. Dabei entsteht zunächst neben der gewünschten Verbindung (VII) auch das o-Toluidin-Hydrochlorid, das sich gewünschtenfalls durch weiteres Erhitzen vollständig zum Amid umsetzen läßt. Der Fortgang der Reaktion läßt sich über die Entstchung von Chlorwasserstoff verfolgen. Zur Vernichtung eventuell verbleibender Reste Buttersäurechlorid kann Methanol nachgesetzt werden. Nach Abkühlen der Reaktionslösung fällt das Amid (VII) aus und läßt sich z.B. durch Filtration mit einer Reinheit von im allgemeinen über 98 % und in Ausbeuten von im allgemeinen 92 bis 95 % isolieren.

Die zweite Stufe des erfindungsgemäßen Verfahrens, die Bromierung der Verbindung (VII) zu Verbindung (VIII), kann beispielsweise durchgeführt werden, indem man die Verbindung (VII) in Essigsäure vorlegt, bei 10 bis 80°C die 1 bis 1,3 molare Menge elementares Brom zusammen mit weiterer Essigsäure zufügt, das Gemisch bei 10 bis 80°C 20 Minuten bis 3 Stunden nachrührt, danach die 0,5 bis 5-fache Volumenmenge Wasser zufügt, den nunmehr vorliegenden Niederschlag abtrennt, mit Wasser wäscht und im Vakuum trocknet. Man kann so die Verbindung (VIII), d.h. N-(4-Brom-2-methylphenyl)-butanamid, in Ausbeuten von im allgemeinen über 95 % und in Reinheiten von im allgemeinen über 99 % erhalten.

Die dritte Stufe des erfindungsgemäßen Verfahrens, die Überführung der Verbindung (VIII) durch Umsetzung mit Kohlenmonoxid und Methanol in Gegenwart eines Palladiumkatalysators zur Verbindung (II), kann beispielsweise durchgeführt werden, indem man die Verbindung der Formel (VIII) und einen Palladiumkatalysator in einem Druckgefäß vorlegt, danach ein Gemisch aus Methanol, gegebenenfalls einen oder mehreren von Methanol verschiedenen Lösungsmitteln und einer Base zufügt, dann bei 90 bis 160°C 2 bis 30 bar Kohlenmonoxid aufdrückt und diesen Druck aufrecht erhält, bis kein Kohlenmonoxid mehr aufgenommen wird.

In der dritten Stufe des erfindungsgemäßen Verfahren kann Methanol als Reaktionspartner und als Lösungsmittel dienen. Man kann gegebenenfalls zusätzlich ein oder mehrere von Methanol verschiedene organische Lösungsmittel einsetzen. Bevorzugte zusätzliche organische Lösungsmittel sind Kohlenwasserstoffe wie Hexan, Cyclohexan, Heptan, Benzol, Toluol, die isomeren Xylole und deren Mischungen, Chlorkohlenwasserstoffe wie Chlorbenzol, Dichlorbenzol, Methylenchlorid und Hexachlorethan, Nitrile wie Acetonitril, Amide wie Dimethylformamid und Ether wie Dioxan und Tetrahydrofuran. Die Verwendung solcher Lösungsmittel ist vorteilhaft, wenn dadurch die Löslichkeit des Kohlenmonoxids in der Lösung erhöht wird. Dann kann diese Reaktionsstufe bei relativ niedrigeren Drucken durchgeführt werden, was vor allem im technischen Maßstab mit weniger apparativem und sicherheitstechnischen Aufwand verbunden ist.

Als Palladiumkatalysatoren kommen z.B. solche des Typs Pd(P Ph₃)₂X₂ mit Ph = gegebenenfalls substituiertem Phenyl und X = Halogen in Frage, die man auch in situ aus PdX₂ und PPh₃ herstellen kann. Die Triphenylphosphinkomponente kann auch im Überschuss eingesetzt werden. Bezogen auf die Verbindung (VIII) kann man beispielsweise 0,1 bis 1 Mol-% Palladiumkatalysator einsetzen.

Als Basen kommen beispielsweise Carbonate, Hydrogencarbonate und Acetate von Alkalimetallen in Frage. Bevorzugt sind jedoch primäre, sekundäre und tertiäre Amine, insbesondere Tri-C₁-C₁₀-alkylamine. Bezogen auf 1 Mol der Verbindung (VIII) kann man beispielsweise 0,9 bis 5 Mol, vorzugsweise 1,05 bis 2 Mol Base einsetzen.

Mit dem erfindungsgemäßen Verfahren kann die Verbindung (II) in einem nur 3-stufigen Verfahren mit guten Ausbeuten hergestellt und in guten Reinheiten erhalten werden. Die Ausbeute beim erfindungsgemäßen Verfahren beträgt über alle 3 Stufen im allgemeinen 90 bis 95 %. Das ist eine wesentliche Verbesserung der Zugänglichkeit zur Verbindung (II) und zu den aus der Verbindung (II) herstellbaren Antagonisten.

Eine Ausführungsform des erfindungsgemäßen Verfahrens ist eine Synthese von (VIII) ohne Zwischenisolierung der Verbindung (VII). Dies ist technisch von Vorteil, da die Zwischenisolierung einer Verbindung immer zusätzliche Aggregate benötigt, die den Prozeß verlangsamen und in der Regel durch Isolationsverluste und Rückstände, z.B. in Mutterlaugen, die Ausbeute verringern.

Bei dieser Ausführungsform wird zunächst vorgegangen, wie zur Herstellung der Verbindung (VII) beschrieben. Von der durch Amidierung erhaltenen rohen Lösung von (VII) wird nach der Butyrylierung das inerte Lösungsmittel destillativ entfernt. Um verbleibende Reste des inerten Lösungsmittels zu entfernen, kann zur Schmelze der Verbindung (VII) Wasser hinzugefügt und wieder abdestilliert werden. Das so erhaltene rohe (VII) kann dann mit einem zur Bromierung geeigneten Lösungsmittel versetzt werden. Bevorzugt ist dies Essigsäure, Ameisensäure, Propionsäure oder ihre Gemische mit Wasser in allen Verhältnissen sowie verdünnte Mineralsäuren wie Schwefelsäure oder nur Wasser. Ebenfalls möglich ist die Bromierung in einem inerten Lösungsmittel unter Zusatz von Lewis-Säuren, wie z.B. Aluminiumchlorid, Aluminiumbromid, Eisenbromid oder unter Zusatz von elementarem Eisen. Besonders bevorzugt sind Essigsäure sowie Gemische von Essigsäure und Wasser.

Zu dieser Reaktionsmischung wird bei Temperaturen von 10 bis 130°C, bevorzugt 30 und 60°C direkt Brom gegeben. Bezogen auf ein Mol der Verbindung (VII) kann 0,9 bis 1,1 Mol Brom bevorzugt 1 bis 1,05 Mol zugegeben werden.

Aufgrund der hierbei entstehenden Bromwasserstoffsäure kann das eingesetzte wertvolle Brom nur zur Hälfte umgesetzt werden. Diese Ausführungsform sieht daher auch vor, nur 0,45 bis 0,95 Mol Brom auf I Mol der Verbindung (VII) einzusetzen und die weitere Bromierung über eine Reoxidation der Bromwasserstoffsäure mit einem Oxidationsmittel, bevorzugt Wasserstoffperoxid, in einer Menge ergänzend auf 1 Mol durchzuführen.

Man kann die Bromierung auch mit Bromwasserstoffsäure durchführen, nach deren Zugabe ein Oxidationsmittel zugefügt wird. Beispielsweise kann man 0,9 bis 1,1 Mol Bromwasserstoffsäure pro Mol der Verbindung (VII) und eine äquivalente Menge Oxidationsmittel, vorzugsweise Wasserstoffperoxid, verwenden. Gegebenenfalls kann man die Bromwasserstoffsäure als Lösung in Wasser einsetzen. Anstelle von Bromwasserstoffsäure kann man auch Bromide verwenden, vorzugsweise Kaliumbromid. Bromide kann man als solche oder in beispielsweise wässriger Lösung einsetzen.

Technisch problematisch bei allen oben beschriebenen Synthesen ist, dass das Produkt (VIII) bei der Bromierung aufgrund seiner Löslichkeitseigenschaften nach ca. 30 bis 70 % des Reaktionsfortganges spontan und als feiner Feststoff ausfällt. Die dadurch entstehende Suspension läßt sich nur noch schwer rühren. Eine technische Umsetzung ist deshalb nur unter hohem Aufwand durchführbar.

Dieses Problem konnte überraschend gelöst werden, indem die Bromierung in Form einer gleichzeitigen Dosierung - mit einer Toleranz des jeweiligen Vorlaufs eines der Volumenströme von bis zu 20 %, vorzugsweise 10 bis 20 % - einerseits des Broms bzw. des Broms und des Oxidationsmittels und andererseits des rohen (VII) gegebenenfalls einer Mischung aus (VII) und dem zur Bromierung geeigneten Lösungsmittel, durchführt. Die Dosierung kann in eine Vorlage erfolgen, in der ein Teil des zur Bromierung geeigneten Lösungsmittels vorliegt. Auch diese Ausführungsform ist Bestandteil des erfindungsgemäßen Verfahrens. Bevorzugt als Vorlage sind Mischungen von Essigsäure und Wasser im Verhältnis 0,2 zu 0,8 bis 0,8 zu 0,2. Besonders bevorzugt 0,25 zu 0,75 bis 0,5 zu 0,5. Weiterhin wurde das Kristallisationsverhalten verbessert, indem vor der Bromierung zur Vorlage sowie Verbindung (VIII) gegeben wurden (etwas löslich), bis eine Suspension vorlag.

Die Verbindung der Formel (VIII) ist neu. Die vorliegende Erfindung betrifft deshalb auch die Verbindung der Formel (VIII), d.h. N-(4-Brom-2-methylphenyl)-butanamid. Die Herstellbarkeit dieser Verbindung ist weiter oben beschrieben worden. Sie stellt die Schlüsselsubstanz im erfindungsgemäßen Verfahren zur Herstellung der Verbindung der Formel (II) dar. Ihr Auffinden ermöglichte den verbesserten Zugang zur Verbindung der Formel (II) und den daraus herstellbaren Antagonisten.

### Beispiele

### Beispiel 1 Synthese von N-(2-Methylphenyl)-butanamid (VII)

1128,6 g o-Toluidin wurden in 500 ml Toluol vorgelegt und auf 90°C erhitzt. Man ließ 134,3 g Buttersäurechlorid innerhalb von 2 Stunden zutropfen. Nach Dosierende wurde auf Rückfluß erhitzt und bis zum Ende der Gasentwicklung bei dieser Temperatur nachgerührt. Man ließ auf 70°C abkühlen, gab 12 ml Methanol zu und ließ eine Stunde nachrühren. Zur Entfernung des Methanols wurde aufgeheizt und 70 ml abdestilliert. Danach wurden 300 ml Toluol abdestilliert und 400 ml Cyclohexan wieder hinzugefügt. Man ließ auf 10°C abkühlen und erhielt durch Filtration nach dem Trocknen 199,5 g (92 % Ausbeute) des Produktes in 98 % Reinheit.

### Beispiel 2 Synthese von N-(4-Brom-2-methylphenyl)-butanamid (VIII)

30 g N-Butyryl-o-toluidin und 150 g Eisessig wurden bei 30°C vorgelegt. Hierzu wurde unter Rühren eine Lösung von 33 g Brom in 66 g Eisessig zugefügt und anschließend 1 Stunde bei 25 bis 30°C nachgerührt. Zu dem Reaktionsgemisch wurden dann 300 ml Wasser hinzugefügt und nach kurzem Nachrühren filtriert, die Kristalle mit Wasser nachgewaschen und im Vakuum getrocknet. Es wurden 42,5 g (98 % Ausbeute) N-(4-Brom-2-methylphenyl)-butanamid in 99,4 %iger Reinheit erhalten.

| **Physikalische Daten:** | |
|---|---|
| Schmelzpunkt | 146 bis 147°C |
| IR-Spektrum | 3275cm⁻¹, 1649 cm⁻¹ |
| ¹H-NMR-Spektrum bei 400 MHz | δ = 0.93 (t, 3H), 1.62 (qt, 2H), 2.19 (s, 3H), 2.31 (t, 2H), 7.35 (m, 2H), 7.4 (s, 1H), 9.27 (s. 1H). |

### Beispiel 3 Synthese von (N-Butyryl)-4-amino-3-methyl-benzoesäuremethylester (II) ohne zusätzliches Lösungsmittel

In einem Druckautoklaven wurden 90 g N-(4-Brom-2-methylphenyl)-butanamid (erhalten gemäß Beispiel 2), 1,26 g Bis-(triphenylphosphin)-palladium(II)chlorid und 3,78 g Triphenylphosphin vorgelegt. Der Autoklav wurde verschlossen, mit Stickstoff gespült und eine sauerstofffreie Lösung von 78 g Tributylamin in 400 ml Methanol hinzugefügt. Der Autoklav wurde evakuiert, dann wurden 10 bar Kohlenmonoxid aufgedrückt und auf 130°C erhitzt. Der Druck wurde für 4 Stunden auf 14 bar gehalten. Eine HPLC-Analyse (mit externem Standard) des Reaktionsgemisches zeigte die Bildung (N-Butyryl)-4-amino-3-methyl-benzoesäuremethylester in einer Ausbeute von 95 % an.

Danach wurde die Reaktionslösung durch Aufkochen mit Aktivkohle vom Katalysator befreit und anschließend bei 80°C mit 500 ml Wasser versetzt. Gleichzeitig wurde soviel Methanol durch Destillation entfernt wie möglich. Das Produkt fiel aus und wurde durch Filtration isoliert. Man erhielt 73,4 g farblose Kristalle (88 % Ausbeute) in 99,5 %iger Reinheit.

### Beispiel 4 Synthese von (II) mit zusätzlichen Lösungsmitteln

In einem Autoklaven wurden 20 g der Verbindung (VIII) zusammen mit 0,56 g Bis(triphenylphosphin)-palladium(II)chlorid und 3,36 g Triphenylphosphin vorgelegt. Der Autoklav wurde verschlossen und mit Stickstoff gespült. Hierzu wurde eine zuvor entgaste und unter Sauerstoffausschluß gehaltene Lösung aus 21,7 g Tri-nbutylamin, 100 ml Methanol, 100 ml Chlorbenzol und 400 ml Toluol gegeben. Der Autoklav wurde evakuiert und auf 120°C erwärmt. Bei dieser Temperatur wurden 6 bar Kohlenmonoxid aufgedrückt und der Ansatz gerührt bis kein Kohlenmonoxid mehr aufgenommen wurde. Das Reaktionsgemisch wurde nach dem Abkühlen aus dem Autoklav entnommen, vom Katalysator und durch Aufkochen mit Aktivkohle befreit. Eine HPLC-Analyse ergibt einen Umsatz von 84,4 % mit einer Selektivität von 93 %.

### Beispiel 5 Synthese von N-(4-Brom-2-methylphenyl)-butanamid (VIII) über Brom/Wasserstoffperoxid-Bromierung

260 g Toluol und 85,7 g o-Toluidin wurden vorgelegt und die Lösung auf 90°C erwärmt. Bei 85-95°C ließ man 89,5 g Buttersäurechlorid zutropfen. Nach Dosierende wurde der Tropftrichter mit 86 g Toluol nachgespült, dann das Gemisch auf Rückfluß aufgeheizt und bis zum Ende der Gasentwicklung plus einer Stunde Nachreaktionszeit nachgerührt. Danach wurde auf 70-75°C abgekühlt. Es wurden 8 g Methanol zugegeben und eine Stunde bei 75°C gerührt. Zur Entfernung von Methanol und Toluol wurden aufgeheizt (110-130°C) und abdestilliert. Gegen Ende der Destillation wurden 50 g Wasser zugegeben und wieder abdestilliert, um restliches Toluol zu entfernen. Anschließend wurde auf 90°C abgekühlt. Danach wurden 850 g Essigsäure zugegeben und die Lösung auf 50°C abgekühlt. Hierzu ließ man langsam 67,1 g Brom tropfen. Anschließend wurden bei 50°C 28,6 g WasserstoffperoxidLösung zudosiert. Man ließ eine Stunde nachrühren. Dabei fiel das Produkt sehr voluminös und kaum rührbar aus. Die Suspension wurde in 1500 g Wasser eingetragen und der Reaktor cinmalmit 500 g Essigsäure ausgespült. Die Suspension wurde filtriert und mit 2 mal je 500 g Wasser gewaschen. Man erhielt nach dem Trocknen 194,3 g Verbindung (VIII) (95,8 % Ausbeute) mit einer Reinheit von 99,0 %.

### Beispiel 6 Synthese von N-(4-Brom-2-methylphenyl)-butanamid (VIII) über Brom-Bromierung, simultandosiert

a) 3087,5 g Toluol wurden bei 20°C vorgelegt. Hierzu wurden 814,2 g o-Toluidin gegeben. Gleichzeitig wurde die Lösung auf 90°C aufgeheizt. Bei 85-95°C wurden 850,3 g Buttersäurechlorid innerhalb von 2 Stunden zudosiert. Nach Dosierende wurde auf Rückfluß aufgeheizt und bis zum Ende der Gasentwicklung plus eine Stunde Nachreaktionszeit nachgerührt. Danach wurde auf 70-75°C abgekühlt. Es wurden 76 g Methanol zugegeben und eine Stunde bei 75°C gerührt. Zur Entfernung von Methanol und Toluol wurde aufgeheizt (110-130°C) und 2600 g Destillat abdestilliert. Gegen Ende der Destillation wurden 475 g Wasser zugegeben und wieder abdestilliert, um restliches Toluol zu entfernen. Insgesamt wurden 950g Destillat abgenommen. Anschließend wurde auf 90°C abgekühlt. Es folgte die Zugabe von 3000 g Essigsäure, wonach die Reaktionslösung auf 20°C abgekühlt wurde.
b) 3000 g Wasser, 3000 g Essigsäure und 30 g Verbindung (VIII) wurden vorgelegt und auf 50°C erwärmt. Hierzu wurden bei 50°C simultan 1225,5 g Brom und die Essigsäurelösung der Verbindung (VII), erhalten gemäß a), innerhalb von 8 Stunden zudosiert. Man ließ eine Stunde nachrühren. Zu dem Reaktionsgemisch wurden anschließend innerhalb von 2 Stunden 4000 g Wasser zugepumpt, auf 20°C abgekühlt und bei 20°C eine Stunde nachgerührt. Die Suspension wurdeauf einen Filter überführt und abgesaugt. Das Produkt wurde mit 3 x 2375 g Wasser gewaschen. Man erhielt nach dem Trocknen 1755,6 g Verbindung (VIII) (90,2 % Ausbeute) mit einer Reinheit von 99,0 %.

### Beispiel 7 Synthese von N-(4-Brom-2-methylphenyl)butanamid (VIII) über Brom/Wasserstoffperoxid-Bromierung simultandosiert

a) 823,3 g Toluol wurden bei 20°C vorgelegt. Hierzu wurden 217,1 g o-Toluidin gegeben. Gleichzeitig wurde die Lösung auf 90°C aufgeheizt. Bei 85-95°C wurden 226,7 g Buttersäurechlorid innerhalb von 2 Stunden zudosiert. Nach Dosierende wird auf Rückfluß aufgeheizt und bis zum Ende der Gasentwicklung plus eine Stunde Nachreaktionszeit nachgerührt. Danach wurde auf 70-75°C abgekühlt. Es wurden 20 g Methanol zugegeben und 1 Stunde bei 75°C gerührt. Zur Entfernung von Methanol und Toluol wurde aufgeheizt (110-130°C) und 808 g Destillat abdestilliert. Gegen Ende der Destillation wurden 126 g Wasser zugegeben und wieder abdestilliert, um restliches Toluol zu entfernen. Insgesamt wurden 934 g Destillat abgenommen. Anschließend wurde auf 90°C abgekühlt. Es folgte die Zugabe von 400 g Essigsäure, wonach die Reaktionslösung auf 20°C abgekühlt wurde.
b) 933 g Wasser, 666 g Essigsäure und 8 g Verbindung (VIII) wurden vorgelegt und auf 50°C erwärmt. Hierzu wurden bei 50°C sumultan 161,1 g Brom und 50 % der Essigsäurelösung der Verbindung (VII), erhalten gemäß a), innerhalb von 4 Stunden zudosiert. Anschließend wurde bei der gleichen Temperatur 115,2 g 30 %ige Wasserstoffperoxidlösung zudosiert. Nach beendeter Reaktion wurden 1066 g Wasser hinzugefügt und auf 20°C abgekühlt. Nach Filtration und Trocknung erhielt man 462,7 g Verbindung (VIII) (80 % Ausbeute) mit einer Reinheit von 89,6 %.

## Patentansprüche

1. Verfahren zur Herstellung von N-Butyryl-4-amino-3-methyl-benzoesäuremethylester, **dadurch gekennzeichnet, dass** man o-Toluidin mit Buttersäurechlorid zu N-Butyryl-2-methyl-anilin umsetzt, dieses zu N-(4-Brom-2-methylphenyl)-butanamid bromiert und dieses durch Umsetzung mit Kohlenmonoxid und Methanol in Gegenwart eines Palladiumkatalysators in N-Butyryl-4-amino-3-methyl-benzoesäuremethylester überführt.

2. Verfahren nach Anspruch 1, **dadurch gekennzeichnet, dass** man die erste Stufe durchführt, indem man o-Toluidin in einem inerten Lösungsmittel vorlegt und anschließend bei Temperaturen von 50 bis 100°C Buttersäurechlorid zudosiert.

3. Verfahren nach Ansprüchen 1 und 2, **dadurch gekennzeichnet, dass** man die zweite Stufe durchführt, indem man N-Butyryl-2-methyl-anilin in Essigsäure vorlegt, bei 10 bis 80°C die 1 bis 1,3 molare Menge elementares Brom zusammen mit weiterer Essigsäure zufügt, das Gemisch bei 10 bis 80°C 20 Minuten bis 3 Stunden nachrührt, danach die 0,5 bis 5-fache Volumenmenge Wasser zufügt, den vorliegenden Niederschlag abtrennt, mit Wasser wäscht und im Vakuum trocknet.

4. Verfahren nach Ansprüchen 1 bis 3, **dadurch gekennzeichnet, dass** man die dritte Stufe des erfindungsgemäßen Verfahrens durchführt, indem man N-(4-Brom-2-methylphenyl)-butanamid und einen Palladiumkatalysator in einem Druckgefäß vorlegt, danach ein Gemisch aus Methanol, gegebenenfalls einem oder mehreren von Methanol verschiedenen Lösungsmitteln und einer Base zufügt, dann bei 90 bis 160°C 2 bis 30 bar Kohlenmonoxid aufdrückt und diesen Druck aufrecht erhält, bis kein Kohlenmonoxid mehr aufgenommen wird.

5. Verfahren nach Ansprüchen 1 bis 4, **dadurch gekennzeichnet, dass** man als Palladiumkatalysatoren solche des Typs Pd(P Ph₃)₂X₂ mit Ph = gegebenenfalls substituiertem Phenyl und X = Halogen einsetzt.

6. Verfahren nach Ansprüchen 1 bis 5, **dadurch gekennzeichnet, dass** man in der dritten Stufe eine Base zusetzt.

7. Verfahren zur Herstellung von N-(4-Brom-2-methylphenyl)-butanamid, **dadurch gekennzeichnet, dass** man o-Toluidin in einem inerten organischen Lösungsmittel vorlegt, anschließend bei Temperaturen von 50 bis 100°C Buttersäurechlorid zudosiert, das Lösungsmittel entfernt, indem man zur Schmelze des erhaltenen Amids Wasser hinzufügt und wieder abdestilliert, das so erhaltene rohe Amid mit einem zur Bromierung geeigneten Lösungsmittel versetzt und zu dieser Reaktionsmischung bei Temperaturen von 10 bis 130°C 0,45 bis 0,95 Brom pro Mol des Amids und ergänzend auf 1 Mol ein Oxidationsmittel zufügt.

8. Verfahren nach Anspruch 7, **dadurch gekennzeichnet, dass** man die Bromierung durchführt, indem man das Brom und das Oxidationsmittel einerseits und das Amid andererseits mit einer Toleranz des jeweiligen Verlaufs einer dieser Volumenströme von bis zu 20 % gleichzeitig dosiert.

9. N-(4-Brom-2-methylphenyl)-butanamid der Formel

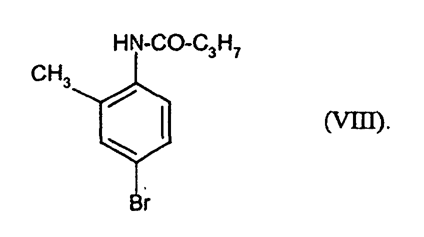

## Claims

1. Process for preparing methyl N-butyryl-4-amino-3-methylbenzoate, **characterized in that** o-toluidine is reacted with butyryl chloride to give N-butyryl-2-methylaniline, the latter is brominated to give N-(4-bromo-2-methylphenyl)butanamide and this is converted by reaction with carbon monoxide and methanol in the presence of a palladium catalyst to give methyl N-butyryl-4-amino-3-methylbenzoate.

2. Process according to Claim 1, **characterized in that** the first stage is carried out by initially charging o-toluidine in an inert solvent and then metering in butyryl chloride at temperatures of from 50 to 100°C.

3. Process according to Claims 1 and 2, **characterized in that** the second stage is carried out by initially charging N-butyryl-2-methylaniline in acetic acid, adding from 1 to 1.3 molar quantity of elemental bromine together with further acetic acid at from 10 to 80°C, continuing to stir the mixture at from 10 to 80°C for from 20 minutes to 3 hours, then adding a water quantity of from 0.5 to 5 times the volume, removing the precipitate formed, washing it with water and drying it under reduced pressure.

4. Process according to Claims 1 to 3, **characterized in that** the third step of the process according to the invention is carried out by initially charging N-(4-bromo-2-methylphenyl)butanamide and a palladium catalyst into a pressure vessel, then adding a mixture of methanol, optionally one or more solvents other than methanol and a base, then pressurizing at from 90 to 160°C to 2-30 bar of carbon monoxide and maintaining this pressure until no more carbon monoxide is taken up.

5. Process according to Claims 1 to 4, **characterized in that** the palladium catalysts used are those of the Pd(P Ph₃)₂X₂ type where Ph = optionally substituted phenyl and X = halogen.

6. Process according to Claims 1 to 5, **characterized in that** a base is added in the third stage.

7. Process for preparing N-(4-bromo-2-methylphenyl)butanamide, **characterized in that** o-toluidine is initially charged in an inert organic solvent, butyryl chloride is then metered in at temperatures of from 50 to 100°C, the solvent is removed by adding water to the melt of the amide obtained and distilling it off again, admixing the crude amide thus obtained with a solvent suitable for bromination and adding from 0.45 to 0.95 of bromine per mole of the amide at temperatures of from 10 to 130°C and, to supplement to 1 mol, an oxidant.

8. Process according to Claim 7, **characterized in that** the bromination is carried out by simultaneously metering in the bromine and the oxidant on the one hand and the amide on the other hand with a tolerance of up to 20% in the respective volume flow rates.

9. N-(4-Bromo-2-methylphenyl)butanamide of the formula

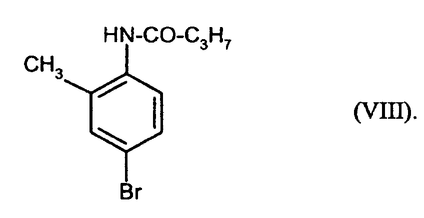

## Revendications

1. Procédé de préparation d'ester de méthyle d'acide N-butyryl-4-amino-3-méthyl-benzoïque, **caractérisé en ce que** l'on transforme, avec du chlorure d'acide butyrique, de l'o-toluidine en N-butyryl-2-méthyl-aniline, que l'on brome celle-ci en N-(4-bromo-2-méthylphényl)-butanamide et que l'on transforme celui-ci en ester de méthyle d'acide N-butyryl-4-amino-3-méthylbenzoïque, par réaction avec du monoxyde de carbone et du méthanol, en présence d'un catalyseur à base de palladium.

2. Procédé selon la revendication 1, **caractérisé en ce que** l'on réalise la première étape, en présentant l'o-toluidine dans un solvant inerte et en ajoutant ensuite progressivement du chlorure d'acide butyrique à des températures comprises entre 50 et 100 °C.

3. Procédé selon les revendications 1 et 2, **caractérisé en ce que** l'on réalise la deuxième étape, en présentant la N-butyryl-2-méthyl-aniline dans de l'acide acétique, en ajoutant entre 10 et 80 °C la concentration 1 à 1,3 molaire de brome élémentaire avec un volume supplémentaire d'acide acétique, en poursuivant l'agitation du mélange entre 10 et 80 °C pendant un laps de temps compris entre 20 minutes et 3 heures, en ajoutant ensuite une quantité d'eau en volume de 0,5 à 5 fois, en séparant le précipité formé, en le lavant à l'eau et en le séchant dans le vide.

4. Procédé selon les revendications 1 à 3, **caractérisé en ce que** l'on réalise la troisième étape du procédé conforme à l'invention, en présentant le N-(4-bromo-2-méthylphényl)-butanamide et un catalyseur à base de palladium dans un récipient de pression, en ajoutant ensuite un mélange de méthanol, le cas échéant d'un ou de plusieurs solvants différents du méthanol et d'une base, en appliquant ensuite une pression de 2 à 30 bar de monoxyde de carbone entre 90 et 160 °C, et en maintenant cette pression jusqu'à ce qu'il ne soit plus absorbé de monoxyde de carbone.

5. Procédé selon les revendications 1 à 4, **caractérisé en ce que** l'on utilise comme catalyseurs à base de palladium ceux du type Pd(P Ph₃)₂X₂ avec pH = un phényle éventuellement substitué et X = un halogène.

6. Procédé selon les revendications 1 à 5, **caractérisé en ce que** l'on ajoute une base au cours de la troisième étape.

7. Procédé de préparation de N-(4-bromo-2-méthylphényl)-butanamide, **caractérisé en ce que** l'on présente l'o-toluidine dans un solvant organique inerte, on ajoute ensuite progressivement du chlorure d'acide butyrique à des températures comprises entre 50 et 100 °C, on élimine le solvant en ajoutant de l'eau à la masse fondue de l'amide obtenu et on réalise une nouvelle séparation par distillation, on mélange l'amide brut ainsi obtenu avec un solvant apte à la bromation et on ajoute à ce mélange réactionnel, à des températures comprises entre 10 et 130 °C, 0,45 à 0,95 mole de brome par mole de l'amide et un oxydant en complément pour obtenir 1 mole.

8. Procédé selon la revendication 7, **caractérisé en ce que** l'on réalise la bromation, en dosant simultanément le brome et l'oxydant, d'une part et l'amide, d'autre part, avec une tolérance du déroulement respectif d'un de ces flux volumiques de 20 % au maximum.

9. N-(4-bromo-2-méthylphényl)-butanamide de la formule
